# EUROPEAN PATENT APPLICATION

(11) **EP 4 555 912 A1**
(43) Date of publication of application: **21.05.2025**
(21) Application number: 23839356.5
(22) Date of filing: 05.06.2023
(51) Int. Cl.: A61B 1/00, G02B 23/24, G02B 23/26

(54) **DISPOSABLE ENDOSCOPE USING PLASTIC GRADIENT-INDEX LENS**

(30) Priority: 11.07.2022 JP 2022111249
(71) Applicant: Koike, Yasuhiro, Kanagawa 225-0012 (JP)
(72) Inventor: KOIKE, Yasuhiro, Yokohama-shi, Kanagawa 225-0012 (JP); KOIKE, Ikko, Yokohama-shi, Kanagawa 225-0012 (JP)
(74) Representative: Schäfer, Matthias W.
(86) International application number: PCT/JP2023/020777
(87) International publication number: WO 2024/014180

(57) **Abstract**

Provided is a disposable endoscope comprising an optical transmission body in a cylindrical sheath having an outer diameter of 20 mm or less, wherein the optical transmission body includes a rod-shaped plastic gradient-index lens and a connector that is detachably connected to a detection unit at a proximal side end portion of the plastic gradient-index lens, and the optical transmission body transmits light received at its distal side end portion to the detection unit, thereby generating an image at the detection unit. This disposable endoscope can obtain high-resolution images and can be disposable, despite using inexpensive materials with a smaller diameter.

## Description

### Cross Reference

This application claims priority to Japanese Patent Application No. 2022-111249 filed on July 11, 2022, and all contents of the application are incorporated herein by reference in their entirety.

### Technical Field

The present invention relates to a disposable endoscope using a plastic gradient-index lens.

### Background Art

Endoscopes allow visual inspection of structures inside cavities. In the medical field, the use of endoscopes allows for the inspection of organs for diagnostic purposes, visualization of surgical sites, sampling of tissues, or safe handling of other surgical instruments.

Generally, rigid endoscopes are configured such that an image obtained at its distal end is transmitted through a relay lens to the inside of the main body provided at its proximal end and the image is observed through an eyepiece provided inside the main body. Here, the distal end is an end portion of the endoscope furthest from the doctor's hand, and the proximal end is an end portion of the endoscope closest to the doctor's hand. Here, the relay lens is understood to mean an optical system that creates at least one one-to-one image. In an endoscope, several relay lenses are combined to form one transmission system with multiple image inversions corresponding to the number of image inversions. In order to clearly observe the image transmitted through the relay lens, the focus needs to be adjusted by finely adjusting the eyepiece frame provided inside the main body in the optical axis direction. In inversion systems that are actually used, the formed image surface exhibits curvature or warping.

In order to construct an optical transmission system consisting of an inversion system with extremely small image surface curvature through a simple design, there is disclosed an endoscope with at least one inversion system with a non-uniform refractive index (see, for example, Patent Literature 1). There is also known an image guide that is composed of a large number of optical fibers bundled together and is used to detect and transmit images in the fields of medical and industrial applications. There is also disclosed a flexible endoscope and an endoscope system including the same that can reduce damage to the fibers that constitute such an image guide and a light guide, and that can be easily connected to a repeater device (see, for example, Patent Literature 2).

On the other hand, there are known a resin rod lens as an optical transmission body for image transmission, and a method for manufacturing the same. The resin rod lens is a so-called graded index (GI) type, and has a continuous refractive index distribution. However, in using rod lenses, if one pitch of the lens is short, a plurality of pitches are required to obtain the required length of the medical image guide. If the number of pitches of the rod lens increases, aberration increases and resolution decreases. For this reason, there is a proposal of using rod lenses with one pitch length of 30 cm or more (see, for example, Patent Literature 3).

### Citation List

### Patent Literature

Patent Literature 1: International Publication No. WO 1998/004949
Patent Literature 2: International Publication No. WO 2018/009971
Patent Literature 3: Japanese Patent Laid-Open No. 2003-185806

### Summary of Invention

### Technical Problem

Patent Literature 1 uses a gradient-index rod lens, indeed. However, there are problems in which the rod lens is made of glass and is easily broken and expensive, the rod lens must be combined with other lenses to form a relay lens system, and rod lens has chromatic aberration that must be corrected. The image guide disclosed in Patent Literature 2 is a bundle of several thousand fine optical fibers with a diameter of several mm (micrometers). Each optical fiber in this optical fiber bundle transmits light incident on one end surface to the other end surface, so that an image on one end surface can be transmitted to the other end surface. However, in such an image guide fiber, a large number of optical fibers must be bundled so that the arrangement is consistent at both end surfaces. In addition, since each of the fibers only transmits a color and a collection of the fibers reproduces the image, a certain number of fibers are required to obtain image information, i.e., resolution. For example, even if 10,000 optical fibers are stacked, only 10,000 pixels can be obtained, which is not necessarily a satisfactory resolution. In addition, the rod lens disclosed in Patent Literature 3 is a lens with a pitch of 30 cm or more indeed, but there is a problem in which the outer diameter of the lens is 10 to 30 mm and this makes it difficult for the endoscope to have a smaller diameter.

The present invention aims to provide a disposable endoscope that can obtain high-resolution images using an inexpensive material with a smaller diameter.

### Solution to Problem

The present invention has been made to solve such problems, and is based on a discovery that gradient-index plastic optical fiber (GI-POF), which has been known as a high-speed optical communication fiber, is capable of transmitting high-definition images. In other words, the present invention includes the following embodiments.
(1) A disposable endoscope including an optical transmission body in a cylindrical sheath having an outer diameter of 20 mm or less, wherein the optical transmission body includes a rod-shaped plastic gradient-index lens (hereinafter, this may be referred to as a "GI-POF lens") and a connector that is detachably connected to a detection unit at a proximal side end portion of the plastic gradient-index lens, and the optical transmission body transmits light received at its distal side end portion to the detection unit, thereby generating an image at the detection unit.
(2) The disposable endoscope of (1), further including an objective lens placed in close contact with or spaced apart from a distal side end portion of the plastic gradient-index lens.
(3) The disposable endoscope of (1) or (2), wherein the sheath is made of a transparent material, the sheath being connected to a light source for illumination at its proximal end portion.
(4) The disposable endoscope of (1) or (2), wherein the sheath includes an outer tube and an illumination optical fiber.
(5) The disposable endoscope of (1) or (2), wherein the plastic gradient-index lens has an outer diameter of 0.1 to 10 mm.
(6) The disposable endoscope of (1) or (2), wherein the plastic gradient-index lens has a length of 1 to 40 cm.
(7) The disposable endoscope of (1) or (2), wherein the sheath is a 10G to 30G injection needle or the sheath is a flexible outer cylinder of the injection needle.
(8) The disposable endoscope of (1) or (2), wherein the plastic gradient-index lens is a plastic lens having an outer diameter of 10 mm or less and a length of 1 to 40 cm, the plastic lens being made of a linear polymer for a matrix, the linear polymer having a dopant added thereto, the dopant being a low molecular weight substance that is not reactive with the polymer, diffusion of the dopant forming a concentration gradient of the dopant in a radial direction in a concentric shape.
(9) The disposable endoscope of (2), wherein the objective lens is a spherical lens.

### Advantageous Effects of Invention

The present invention can provide a disposable endoscope that can obtain high-resolution images despite using an inexpensive material with a smaller diameter.

### Brief Description of Drawings

[Figure 1] Figure 1 is a diagram showing an overall configuration of an endoscope of a first embodiment of the present invention.
[Figure 2] Figure 2 is a partial cross-sectional view of the endoscope showing a state in which an optical transmission body is inserted into a sheath.
[Figure 3] Figure 3 is a partial cross-sectional view of an endoscope having a sheath of another embodiment.
[Figure 4] Figure 4 is a side view of an optical transmission body of one embodiment.
[Figure 5] Figure 5 is a side view of an endoscope front end portion of another embodiment.
[Figure 6] Figure 6 is a cross-sectional view taken along a line A-A' in Figure 2.
[Figure 7] Figure 7 is a schematic diagram for explaining a method of using an endoscope.
[Figure 8] Figure 8 is a schematic diagram showing light rays passing through a GI-POF lens.
[Figure 9] Figure 9 is a diagram showing an overall configuration of an endoscope of a second embodiment of the present invention.
[Figure 10] Figure 10 is a front view of the endoscope of Figure 9.
[Figure 11] Figure 11 is a cross-sectional view taken along a line B-B**'** shown in Figure 10.

### Description of Embodiments

Embodiments of the present invention will be described with reference to the drawings. Note that each embodiment described below does not limit the invention of the claims, and not all of the elements and combinations described in each embodiment are essential to the solution of the present invention.

### (First embodiment)

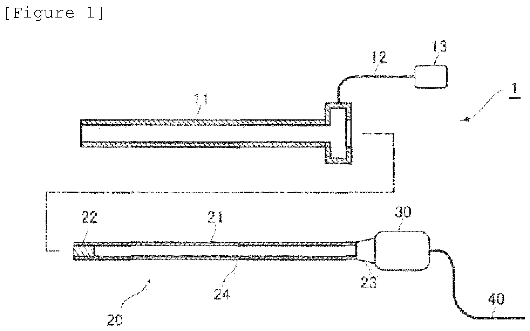

Figure 1 is an overall configuration diagram of an endoscope 1 of a first embodiment of the present invention. Endoscopes are devices for observing affected areas inside bodies, and include, for example, vascular endoscopes, digestive endoscopes, and abdominal endoscopes. Endoscopes for joint diseases are used to diagnose wear on the joint sliding surfaces, formation of osteophytes, ligament ruptures, and meniscus damage that are objects to be observed. The endoscopes are preferable in that they allow simple and rapid diagnosis through direct observation of the affected areas, rather than indirect diagnosis with X-rays, CT, or MRI. Or it is now possible to observe parts that could not be observed before, such as nerve tissue in the spinal cord. Apparatuses for observing and illuminating a patient's body surface from a location away from the patient's body are also known, and such devices are called also "exoscopes." This makes it easier to illuminate and observe, for example, the surfaces of teeth in the oral cavity or the interior of the ear canal, so that diagnosis and surgery performed by doctors are made easier. Connecting a video camera allows an image of the surgical field to be displayed on a screen, so that the surgeon and, in some cases, additional people can observe the image without getting tired. Therefore, the use of an "endoscope" herein also includes observing such body surfaces. Note that the word "disposable" herein means "formed inexpensively to the extent of being disposable within a practical range", and does not mean "must be thrown away after each use."

The endoscope 1 shown in Figure 1 is used to observe any location of a living body including joints (wear on the joint sliding surfaces, formation of osteophytes, ligament ruptures, etc.), the spine, spinal cord, eyes, ears, nose and throat, urinary organs, etc. (hereinafter collectively referred to as the "affected areas"), which are objects to be observed. The endoscope 1 includes a sheath 11, an illumination optical fiber 12, a light source 13, an optical transmission body 20 consisting of a GI-POF lens 21 and an objective lens 22 as necessary, and a connector 23. The connector 23 is detachably connected to the detection unit 30. In Figure 1, an outer cylinder 24 made of metal or plastic is provided on the outside of the optical transmission body 20. The outside of the GI-POF lens 21 may be painted to block light, or the inside of the outer cylinder 24 may be painted black to prevent stray light. The detection unit 30 may include a CCD camera or a CMOS image forming device. The detection unit 30 may also be connected to a monitor (not shown) via an electric cable or an optical fiber cable 40 so that the monitor displays an image generated by the detection unit 30.

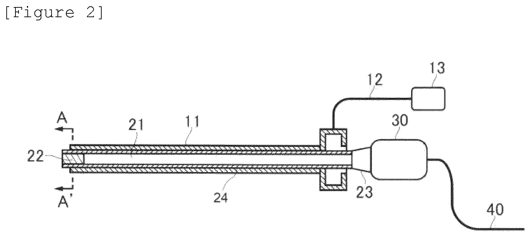

Figure 2 is a partial cross-sectional view of the endoscope showing a state in which the optical transmission body 20 is inserted into the sheath 11. The sheath herein means a short catheter that is placed inside the body. The sheath 11 covers the optical transmission body 20, is elongated (semi-) transparent hollow (tube) with openings at both the front and rear ends, is flexible, and is integrally formed from a plastic material (resin material). For example, a thermoplastic resin is used as the plastic material. The thermoplastic resins are preferably polytetrafluoroethylene (PTFE), ethylene-tetrafluoroethylene copolymer (ETFE), polyurethane (PU), tetrafluoroethylene-perfluoroalkylvinyl ether copolymer (PFA), polyurethane (PUR), polypropylene (PP), polyethylene (PE), polyvinyl chloride (PVC), acrylonitrile-butadiene-styrene copolymer, polycarbonate, polyamide, polyoxymethylene, etc. Of these, PTFE, ETFE, PP, and PU are preferred.

In a preferred embodiment, the sheath 11 may be made of a transparent material and have the function of transmitting light of the light source 13 with which the affected area is irradiated. For example, in Figure 2, light from the light source 13 is guided to the sheath through the illumination optical fiber 12. The optical fiber 12 may pass through the inside of the resin that constitutes the sheath 11 to the distal end of the sheath 11. In this case, the sheath does not need to be transparent and may be made of a high-strength material such as metal. In Figure 2, the optical transmission body 20 is used with its front end portion protruding from the front end portion of the sheath 11. An objective lens 22 is provided on the front end surface of the optical transmission body 20. The rear end of the optical transmission body 20 is connected to the connector 23. The detailed internal structure of the optical transmission body 20 will be described later, but the sheath 11 has an outer diameter of 20 mm or less to cover the optical transmission body 20, and preferably 10 mm or less, and more preferably 5 mm or less.

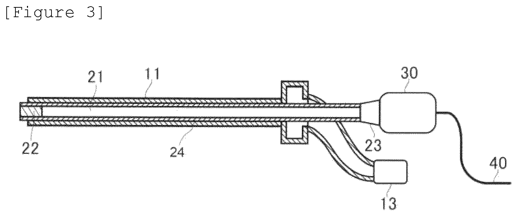

Figure 3 is a partial cross-sectional view of an endoscope having a sheath 11 in another embodiment. In this embodiment, the proximal end of the sheath 11 is directly connected to the light source 13, and the distal end is irradiated with light from the light source 13 through the inside of the sheath 11. At this time, there may be a gap between the sheath 11 and the optical transmission body 20. The distal end is irradiated with the light from the light source 13 through the gap or through within the transparent resin that constitutes the sheath 11. The proximal end of the sheath 11 may have a hole through which the optical transmission body 20 passes. In addition, the gap between the sheath 11 and the optical transmission body 20 may have a space for passing water to clean the affected area or a tube for passing a treatment tool such as forceps.

The connector 23 is cylindrical, and the optical transmission body 20 is fixed to the front end portion of the connector 23. An imaging element such as a CCD camera is installed inside the connector 23 or the detection unit 30 via the connection portion of the optical transmission body 20. The inside of the connector 23 or the detection unit 30 may also be provided with an imaging lens for forming an image of the end surface of the GI-POF lens 21 on the imaging element. The rear end portion of the connector 23 is detachably connected to the detection unit 30 via an appropriate connection structure such as screwing or fitting.

The CCD camera forms an image of the light from the affected area incident from the optical transmission body 20 on the light receiving surface, and converts the obtained optical image into an electrical signal.

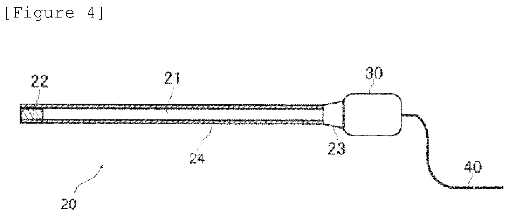

Figure 4 is a side view of the optical transmission body 20 of one embodiment. In this embodiment, the optical transmission body 20 includes a plastic gradient-index lens (GI-POF lens) 21. A plastic gradient-index lens is a plastic lens that has a lens effect due to a refractive index distribution. The plastic gradient-index lens is basically the same as a GI-type plastic optical fiber, but differs in that the optical signal is not recognized as digital data but can be recognized by the human eye as an image. The lens is more preferably used as a medical endoscope as the outer diameter of the lens is smaller. However, as the outer diameter of the lens, i.e., the radius of the optical transmission path, decreases, the length of one pitch of the lens also decreases, and a plurality of pitches are required to obtain the required length as a medical image guide. As a result, there is a problem in which the aberration increases and the resolution decreases. For this reason, the outer diameter of the plastic gradient-index lens is preferably 0.1 to 10 mm, more preferably 0.1 to 5 mm, even more preferably 0.1 to 1 mm, and even more preferably 0.1 to 0.5 mm. Furthermore, for use as an endoscope, the optical transmission body 20 needs to have a certain length, and the normal length of the plastic gradient-index lens is 1 to 40 cm, preferably 1 to 20 cm, more preferably 1 to 10 cm, and even more preferably 1 to 5 cm.

In this embodiment, an objective lens 22 is provided at the front end on the distal end side of the optical transmission body 20 in close contact with the GI-POF lens 21. In order to capture the light spreading from the object over a wide angle, the objective lens 22 desirably has a large numerical aperture (NA) and an appropriate field number and depth of field. For example, an objective lens with a field angle of 150° to 170° has been reported as such an objective lens (see Japanese Patent Laid-Open No. 2006-251272).

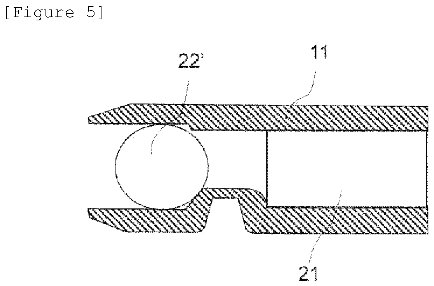

Figure 5 is a side view of the endoscope front end portion of another embodiment. In this embodiment, the objective lens 22' is a ball lens (spherical lens). The ball lens protects the end surface of the GI-POF lens 21 and can condense the parallel light entering the ball lens 22' onto the GI-POF lens 21. This is because, like the objective lens 22 shown in Figure 4, the ball lens 22' has a convex lens function and therefore acts as an objective lens that widens the field angle. In addition, the distance between the ball lens 22' and the GI-POF lens 21 can be controlled with high accuracy. The structure of the endoscope front end portion in this embodiment is such that a spherical lens is attached to the GI-POF lens using ballpoint pen technology. This achieves highly accurate attachment of the spherical lens at low cost.

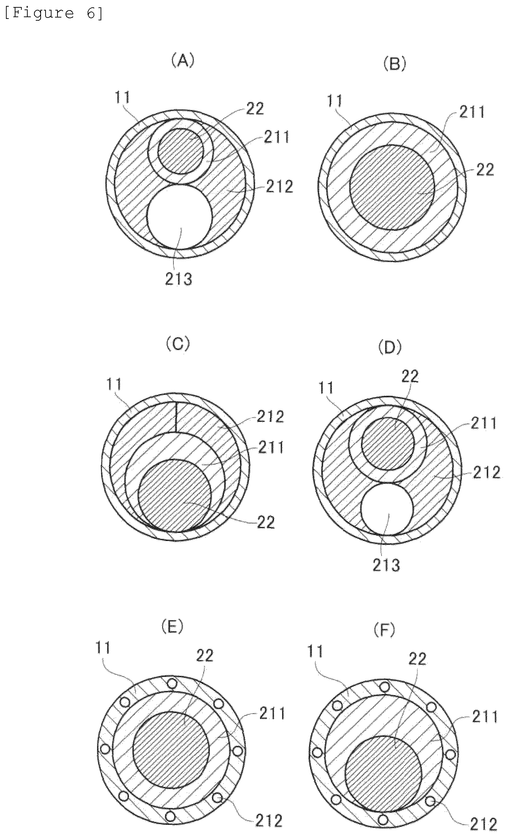

Figure 6 are diagrams each showing a variation in the placement of the optical transmission portion and the illumination portion of the endoscope, is a cross-sectional view taken along a line A-A' shown in Figure 2, and is a schematic diagram in which the sheath 11 and the outer cylinder 24 are depicted as a single unit as a sheath 11. As shown in Figures 6(A) to 6(F), the front end of the optical transmission body 20 includes an objective lens 22 and an illumination optical fiber 211. The illumination optical fiber 211 is a bundle of many thin optical fibers covered with a cylindrical coating layer. The illumination optical fiber 211 covers the circumference of the objective lens 22, and transmits light from the light source 13 (see Figure 1) with which the affected area is irradiated. In the embodiment shown in Figure 6, since the light source is included in the optical transmission body, the sheath 11 and the outer cylinder 24 do not need to transmit light from the light source and may be integrally made of a metallic material. The objective lens 22 may be, for example, a combination lens or a ball lens with a wide field angle as described above. The GI-POF lens 21 transmits the light from the affected area received from the objective lens 22. As shown in Figures 6(A) and 6(D), the optical transmission body 20 may have a space (hole) 212 for allowing water to pass therethrough to clean the affected area and a tube 213 for allowing a treatment tool such as forceps to pass therethrough. The size and shape of each of these tubes can be changed depending on the purpose of use. Alternatively, as shown in Figures 6(E) and 6(F), for example, holes 212 for water supply and drainage may be provided in the metal sheath 11. Note that in Figure 6, the objective lens 22 is attached to the front end of the general optical transmission body 20 described in Figure 2, but this is not necessarily required, and the GI-POF lens 21 may be exposed at the front end instead of the objective lens 22.

### (Method of using an endoscope)

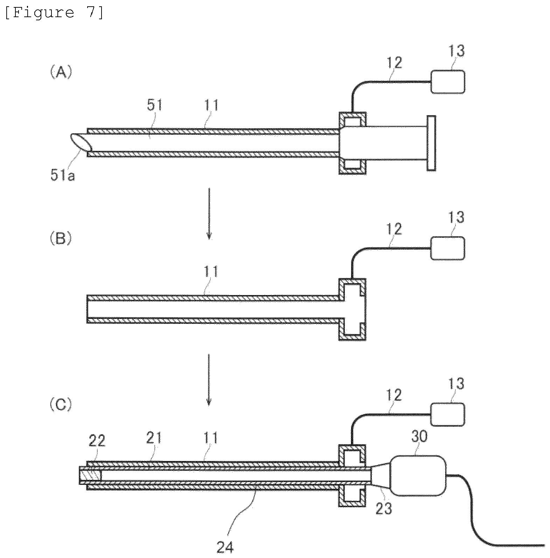

Next, a method of using the endoscope 1 of the first embodiment will be described with reference to Figure 7. As shown in Figure 7(A), the injection needle 51 covered with the sheath 11 as an outer cylinder is inserted into the affected area. In other words, the puncture portion 51a of the injection needle 51 is inserted into the affected area together with the sheath 11. The injection needle 51 may be, for example, an 18G injection needle. The injection needle 51 is inserted into the affected area and has a function of guiding the sheath 11 to the affected area. The injection needle 51 is cylindrical with an outer diameter of about 1.2 mm, and the front end portion is cut obliquely relative to the axial direction to form the piercing portion 51a. The piercing portion 51a pierces the patient's skin to insert the injection needle 51 into the patient's body, and thereby the front end portion of the sheath 11 approaches the affected area. Note that, since the injection needle 51 is thin, the patient feels little pain when the injection needle 51 is inserted into the patient's body, and no anesthesia is required for the patient when the injection needle 51 is inserted into the patient's body. In addition, since there is no need to suture the wound caused by the injection needle 51, the endoscope can be used in clinics that do not have operating rooms. In order for the endoscope to achieve such actions and effects, the diameter of the injection needle 51 to be used can be about 0.3 mm (30 G) to 3.4 mm (10 G), and preferably about 0.6 mm (24 G) to 1.2 mm (18 G).

In this state, the injection needle 51 is removed leaving the sheath 11, and the sheath 11 is placed in the affected area (Figure 7(B)). Next, as shown in Figure 7(C), the optical transmission body 20 is inserted into the base end of the sheath 11, so that the objective lens 22 approaches the affected area, and the reflected light of the light from the light source 13, with which the affected area is irradiated through the sheath 11, can be received by the objective lens 22 at the front end portion of the optical transmission body 21.

In another embodiment, the endoscope 1 does not need to have the light source 13 and the optical fiber 12 for illumination. For example, the endoscope 1 inserted into the affected area of the knee joint may radiate light from the back side of the affected area to illuminate the inside of the knee joint, and may transmit the light captured by the objective lens 22 to the detection unit 30 through the optical transmission body 20.

### (Method for manufacturing plastic gradient-index lens)

A method for manufacturing a plastic gradient-index lens (GI-POF lens) in a preferred embodiment of the present invention is basically the same as the method for manufacturing a GI-type plastic optical fiber, and includes the following steps (a) to (c).
(a) A rod is produced in which a non-reactive dopant is uniformly doped in a linear polymer. This is used as a core rod.
(b) A polymer tube is produced with an inner diameter equal to the outer diameter of the rod. The material of this polymer tube may be the same as that of the linear polymer that normally constitutes the core rod.
(c) The core rod is inserted into the polymer tube and left at a temperature higher than the glass transition temperature of the polymer. At this time, the dopant in the core rod diffuses into the surrounding polymer tube by thermal diffusion. This forms a dopant concentration distribution in the radial direction. Here, if the refractive index of the dopant is higher than that of the polymer, a GI-type (Graded Index) rod is obtained in which the refractive index is highest at the central axis and gradually decreases toward the periphery.

Although the polymer tube is one, it is possible to further add polymer tubes on its outside. The outer polymer tubes each have the same inner diameter as the outer diameter of its inter polymer tube. The purpose of this is to form a more optimal refractive index distribution by increasing the number of polymer tubes. In that case, the outer polymer tubes are doped with a dopant with a lower dopant concentration than the core rod, and the initial dopant concentration decreases toward the peripheral polymer tubes. In other words, the initial distribution before diffusion when multiple polymer tubes are set has a multiple-step distribution. Keeping this at a temperature above the glass transition temperature causes diffusion between adjacent polymer tubes, alleviates the multi-step distribution, and forms a single smooth refractive index distribution.

This process produces a GI-POF preform with a refractive index distribution in the radial direction. The preform is set in a heat stretching device and gradually moved into the heating furnace. The preform is inserted at a speed of V1. Meanwhile, the end of the preform inserted in the heating furnace is stretched at a speed of V2 to produce a GI-type POF with approximately the same refractive index distribution as the preform. The stretching ratio is defined as V2/V1. For example, when the stretching ratio is 100 times, the diameter of the GI-POF is 1/10 of the preform. When a preform with an outer diameter of 25 mm j is subjected to heat stretching at a stretching ratio of 10,000 times, an extremely thin GI-POF with an outer diameter of 0.25 mm is obtained.

The obtained GI-POF is cut to several centimeters and both end surfaces are polished to produce a GI-POF lens that transmits images.

The polymer used in this embodiment is not particularly limited as long as it has a linear structure. Basically, the polymer just needs to be a transparent polymer that maintains an amorphous structure and has the mechanical properties of so-called plastics. Specific examples of candidate of such polymers include, but are not limited to, commercially available general polymers such as various acrylic polymers, polystyrene, and polycarbonate.

As for the dopant, a candidate dopant has a refractive index higher than the polymer, does not aggregate in the linear polymer, and has excellent compatibility. To increase the refractive index of the dopant, candidates are low molecules with high polarizability, such as aromatics (benzene rings), halogen-based substituents other than fluorine, and sulfides. Preferred dopants are low molecular weight compounds and oligomers that contain aromatic rings such as benzene rings, halogen atoms such as chlorine, bromine, and iodine, and bonding groups such as ether bonds. In addition, as a reverse diffusion method, there is also a method in which the dopant is doped in the surrounding tubes and diffuses from the surrounding tubes toward the central rod. In that case, a candidate dopant has a refractive index lower than the polymer.

To increase transparency, the dopant needs to be uniformly dispersed in the polymer. To achieve this, the dopant is preferably a dopant with a solubility parameter close to that of the polymer. If the solubility parameters of the two are different, the low molecular weight compounds will aggregate together and become scatterers, causing cloudiness.

In another embodiment of the method for manufacturing a plastic gradient-index lens, the lens may be made of a copolymer using two types of monomers. For example, a mixed solution of two types of M1 and M2 monomers, in which a polymerization initiator and, if necessary, a chain transfer agent is dissolved, is injected into a polymer tube made of a transparent acrylic polymer or the like. The number of types of monomers may be increased, but for the sake of simplicity, two types are described here. Here, the M1 polymer obtained by polymerizing the M1 monomer has a lower refractive index than the M2 polymer obtained by polymerizing the M2 monomer. For example, methyl methacrylate is a candidate for the M1 monomer, and benzyl methacrylate is a candidate for the M2 monomer.

The polymer tube filled with this monomer solution is heated at an appropriate temperature, or is irradiated with UV from the surroundings, to start polymerization. At this time, the inner wall of the polymer tube (normally made of M1 polymer) is slightly swelled by the mixed monomer solution, and a gel layer is formed. In the gel layer, the polymerization termination reaction is reduced, so that the polymerization speed is accelerated and polymerization occurs preferentially from the gel layer formed on the inner wall of the polymer tube. The M2 monomer has a benzene ring or the like to increase the refractive index, and the M2 monomer tends to gradually gather in the center, whereas the polymer layer is gradually formed by polymerization from the periphery, due to difference in compatibility. Furthermore, when the polymer tube made of the M1 polymer dissolves from the inner wall toward the center, the M2 monomer is pushed toward the center as a result. Therefore, the composition ratio of the M2 monomer unit is low in the copolymer formed near the inner wall of the polymer tube, and the composition ratio of the M2 monomer unit increases toward the center. Since the refractive index of the M2 polymer is higher than that of the M1 polymer, in a preform rod where polymerization has been fully completed, a copolymer is obtained in which the refractive index is highest at the center and gradually decreases toward the periphery. The process of stretching this preform to obtain GI-POF is the same as the manufacturing method described above.

Even if a non-polymerizable M2 dopant is used instead of the M2 monomer, a similar refractive index distribution can be formed by the manufacturing method of this embodiment. The structure in that case is the same as that of the preform through the manufacturing method described above, in which the concentration of unreactive dopant gradually changes in the polymer matrix.

A manufacturing method in yet another embodiment is to manufacture a gradient-index POF by melt extrusion in one process. The core polymer is a polymer in which a dopant with a high refractive index is uniformly dissolved, and the clad polymer is a homopolymer containing no dopant. A simultaneous melt extrusion of these two types of polymers forms a clad layer around the core. A diffusion tube is installed immediately after melt extrusion, and the dopant of the core polymer having undergone the melt extrusion gradually diffuses into the molten clad polymer layer while passing through the diffusion tube. Controlling the melting conditions, such as the diffusion temperature and diffusion speed, makes it possible to produce a GI-POF with a desired refractive index distribution.

The above describes three types of methods for manufacturing GI-POF, but what these manufacturing methods have in common is that the polymer is a linear polymer and can be stretched.

### (Advantages of being a linear polymer)

Other than linear polymers, there are also polymers each having molecular chains with a network structure. A method for manufacturing GI lenses has been reported in which M2 monomers each having a low-refractive index are diffused from the surroundings into a gel rod with a network structure, which is in process of polymerization, to form a refractive index distribution. In this case, the gel rod with a three-dimensional network structure is convenient for maintaining its shape during diffusion. The M2 monomers diffusing from the surroundings copolymerize with the gel rod, and a hard GI rod lens is finally obtained. This lens has polymer chains each having a network structure, so that a thin GI-POF cannot be made through heat stretching, as in the present invention. To subject a preform to heat stretching, the polymer must be a linear polymer. In addition, slight microscopic non-uniform structures and fluctuations may occur during the diffusion of the monomer into the gel rod. These may cause image blurring and light scattering.

In contrast, since the polymer used in the present invention is a linear polymer, the preform can be easily subjected to heat stretching. If a GI preform has a microscopic non-uniform structure and the preform itself is made into a lens, the microscopic non-uniform structure may cause image blurring and light scattering. However, if the preform is stretched at a high stretching ratio of 10,000 times, for example, the microscopic non-uniform structure will be reduced to 1/100 in the radial direction and will be stretched 10,000 times in the stretching direction. This means that the non-uniform structure is essentially negligible compared to the wavelength of light, so that image blurring and light scattering due to the microscopic non-uniform structure is unlikely to occur. Thus, the use of linear polymers is advantageous in implementing the present invention, but the present invention does not necessarily exclude other polymers, and as long as a disposable endoscope has the configuration described in claim 1, it is included in the disposable endoscope of the present invention.

The GI-POF lens used in the present invention is extremely thin and is inserted into an injection needle as a relay lens with a length of several centimeters to several tens of centimeters, and is required to have high-quality image formation characteristics. For this reason, the above-mentioned preform stretching process is an important manufacturing method for the present invention. In addition, once a preform rod is produced, an extremely thin GI-POF lens can be produced efficiently, resulting in low cost and achievement of the first disposable GI endoscope ever.

### (Actions and effects)

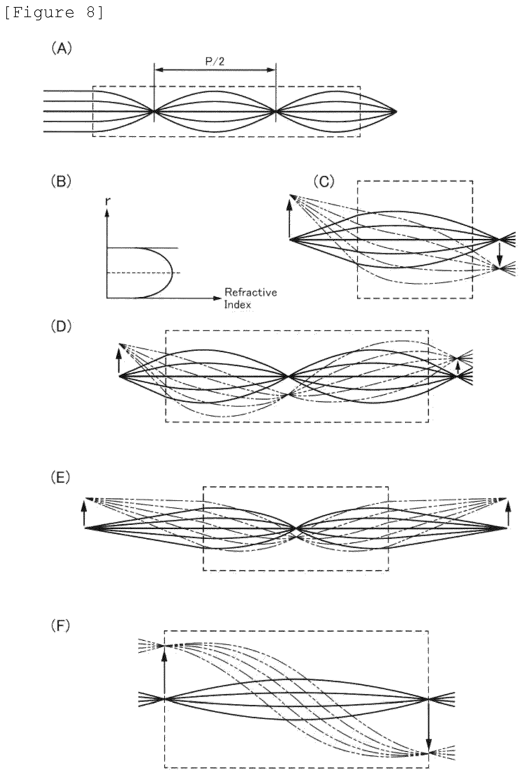

The actions and effects of the present invention will be explained with reference to Figure 8. Figure 8(A) shows light rays passing through a GI-POF lens. Figure 8(B) is a graph showing the relationship between the radial distance r of the GI-POF lens (vertical axis) and the refractive index represented on the horizontal axis. Light rays travel in a straight line in a homogeneous material, and are refracted and bent at the interface between materials with different refractive indices. As shown in Figure 8(B), the refractive index has a radial distribution that is lower toward the periphery from the optical axis (center line). For this reason, the internal light rays travel in a sine wave pattern with a period of the pitch length P, and travel in a sine wave pattern with a period of 1/2 pitch (P/2), repeating image formation every length P/2. In other words, an inverted image is obtained at a 1/2 pitch length, and an erect image is obtained at a 1 pitch length. Figures 8(C) to 8(F) show that the change in lens length can change the focal length. In Figure 8(C) an inverted image is observed, in Figure 8(D) an erect image is observed, and in Figure 8(E) an erect image is observed, each on an image plane at a predetermined distance from the end surface of the lens. In Figure 8(F), it can be seen that an inverted image is formed at the end surface of the lens.

The number of pitches of the GI-POF lens is preferably small in order to obtain a clear image. In order to produce an optical transmission body that can be used as an endoscope, it is sufficient to have about 1 to 10 pitches, preferably about 1 to 5 pitches, and more preferably 2 to 3 pitches.

### (Second embodiment)

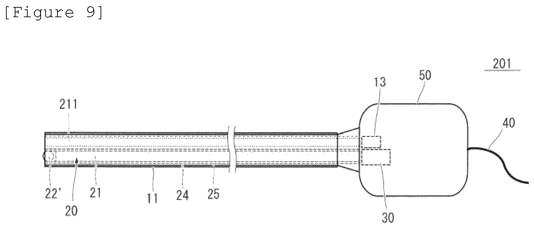

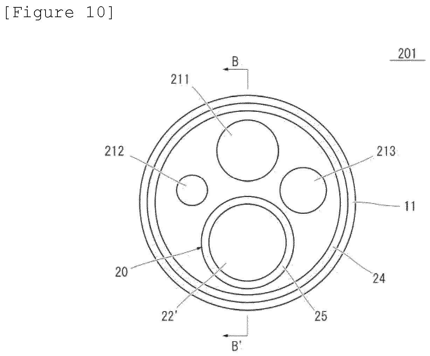

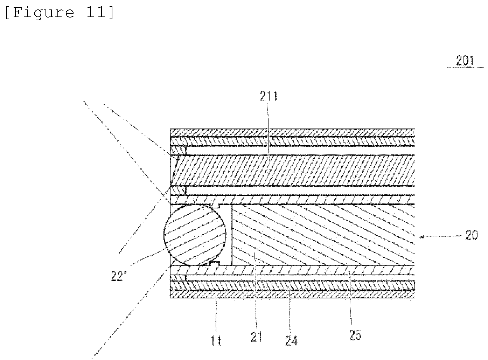

Figure 9 is a diagram showing an overall configuration of an endoscope of a second embodiment of the present invention. Figure 10 is a front view of the endoscope of Figure 9. Figure 11 is a cross-sectional view taken along a line B-B' shown in Figure 10.

The endoscope 201 shown in Figure 9 basically has a configuration with functions similar to those of the endoscope 1 shown in Figure 2, but the placement of the illumination components is different from that of the endoscope 1 in Figure 2. In other words, in the endoscope 1 shown in Figure 2, the light source 13 is configured as a separate body from the detection unit 30, and illumination light from the light source 13 is transmitted to the sheath 11 and radiated from the distal end, whereas the endoscope 201 of the second embodiment shown in Figure 9 is different in that this is not the case. The endoscope 201 of the second embodiment will be described below, but components having the same functions as those in the first embodiment will be denoted by the same reference numerals and characters on the drawings and the description of the functions will be omitted.

As shown in Figure 9, the endoscope 201 includes a sheath 11, an illumination optical fiber 211, a light source 13, an optical transmission body 20, a connector 23, and a grip case 50 that houses the light source 13 and the detection unit 30. The endoscope 201 also includes an outer cylinder 24 made of metal or plastic and surrounding the illumination optical fiber 211 and the optical transmission body 20. The optical transmission body 20 includes a GI-POF lens 21, a ball lens 22' (spherical lens) as an objective lens, and a lens barrel 25 that holds the outer circumferential surfaces of the GI-POF lens 21 and the ball lens 22'. The connector 23 presses the proximal end of the outer cylinder 24 against the grip case 50 and is detachably connected to the grip case 50. As shown in Figure 10, the endoscope 201 has the illumination optical fiber 211 and the optical transmission body 20 inserted side by side inside the outer cylinder 24. The inside of the outer cylinder 24 in the endoscope 201 may also be provided with a space (hole) 212 for allowing water to pass therethrough to clean the affected area and a tube 213 for allowing a treatment tool such as forceps to pass therethrough.

As shown in Figure 11, the endoscope 201 may have the ball lens 22' of the optical transmission body 20 fixed at a position equivalent to or protruding from the distal ends of the outer cylinder 24 and the lens barrel 25. This prevents the distal end of the outer cylinder 24 or that of the lens barrel 25 from blocking the maximum aperture range of the ball lens 22' so that the ball lens 22' can also capture light from its lens circumferential potion away from the optical axis, making it possible to obtain a bright image. Since the endoscope of the present invention uses the GI-POF lens 21 for optical transmission and the incident light does not travel in a straight line, the incident angle at which transmission is possible can be set wide. The endoscope of the present invention is compatible with a lens configuration in which a wide-angle lens such as the ball lens 22' is used and the numerical aperture (NA) is set large. Also in the second embodiment, it is of course possible to use a convex lens, a lens composed of a plurality of lenses, etc., as the objective lens instead of a ball lens.

The grip case 50 is a metal or resin case that houses the light source 13 and the detection unit 30, and is held by the user.

In the endoscope 201 configured in this manner, the outer cylinder 24, inside which the illumination optical fiber 211 and the GI-POF lens 21 are placed, can be separated from the grip case 50 by the connector 23. This allows the portion on the outer cylinder 24 side, which can be formed relatively inexpensively, to be disposable and replaceable.

The endoscope 201 of the second embodiment is a disposable endoscope including an optical transmission body 20, similarly to the endoscope 1 of the first embodiment. The optical transmission body 20 includes a rod-shaped GI-POF lens 21, and a connector 23 that is detachably connected to a detection unit 30 (a grip case 50 that houses the detection unit 30) at the proximal side end portion of the GI-POF lens 21. The GI-POF lens 21 can be formed with a small diameter at low cost, and can transmit images with a relatively high resolution. In the endoscope 201, the optical transmission body 20 transmits light received at its distal side end portion to the detection unit 30, and the light causes the detection unit 30 to generate an image. The endoscope 201 can obtain high-resolution images using the inexpensive material with a smaller diameter, and allows the portion inserted into the body to be disposable.

### Reference Signs List

1, 201 endoscope
11 sheath
12 illumination optical fiber
13 light source
20 optical transmission body
21 GI-POF lens
211 illumination optical fiber
212 space (hole)
213 tube
22 objective lens
23 connector
24 outer cylinder
25 lens barrel
30 detection unit
40 cable
50 grip case

### Industrial Applicability

The endoscope of the present invention can obtain high-resolution images and can be used as a disposable endoscope.

## Claims

1. A disposable endoscope comprising an optical transmission body in a cylindrical sheath having an outer diameter of 20 mm or less, wherein
the optical transmission body comprises a rod-shaped plastic gradient-index lens and a connector detachably connected to a detection unit at a proximal side end portion of the plastic gradient-index lens, and
the optical transmission body transmits light received at its distal side end portion to the detection unit, thereby generating an image at the detection unit.

2. The disposable endoscope of claim 1, further comprising an objective lens placed in close contact with or spaced apart from a distal side end portion of the plastic gradient-index lens.

3. The disposable endoscope of claim 1 or 2, wherein the sheath is made of a transparent material, the sheath being connected to a light source for illumination at its proximal end portion.

4. The disposable endoscope of claim 1 or 2, wherein the sheath includes an outer tube and an illumination optical fiber.

5. The disposable endoscope of claim 1 or 2, wherein the plastic gradient-index lens has an outer diameter of 0.1 to 10 mm.

6. The disposable endoscope of claim 1 or 2, wherein the plastic gradient-index lens has a length of 1 to 40 cm.

7. The disposable endoscope of claim 1 or 2, wherein the sheath is a 10G to 30G injection needle or the sheath is a flexible outer cylinder of the injection needle.

8. The disposable endoscope of claim 1 or 2, wherein the plastic gradient-index lens is a plastic lens having an outer diameter of 10 mm or less and a length of 1 to 40 cm, the plastic lens being made of a linear polymer for a matrix, the linear polymer having a dopant added thereto, the dopant being a low molecular weight substance that is not reactive with the polymer, diffusion of the dopant forming a concentration gradient of the dopant in a radial direction in a concentric shape.

9. The disposable endoscope of claim 2, wherein the objective lens is a spherical lens.
